# EUROPEAN PATENT APPLICATION

(11) **EP 3 375 370 A1**
(43) Date of publication of application: **19.09.2018**
(21) Application number: 17160493.7
(22) Date of filing: 13.03.2017
(51) Int. Cl.: A61B 5/103, A61B 5/11, A61B 5/00, B26B 19/38

(54) **AN APPARATUS AND A METHOD OF ESTIMATING THE POSITION OF A HANDHELD PERSONAL CARE DEVICE RELATIVE TO A USER**

(71) Applicant: Koninklijke Philips N.V., 5656 AE Eindhoven (NL)
(72) Inventor: Jurna, Martin, 5656 AE Eindhoven (NL); Palero, Jonathan, 5656 AE Eindhoven (NL); Visweswara, Ashoka Sathanur, 5656 AE Eindhoven (NL); Buil, Vincent, 5656 AE Eindhoven (NL)
(74) Representative: de Haan, Poul Erik

(57) **Abstract**

There is provided a method of estimating a position of a handheld personal care device relative to a user of the handheld personal care device, the method comprising obtaining one or both of (i) measurements of a weight distribution of the user in a predetermined area over time and (ii) measurements of a torque imparted to the predetermined area by one or both feet of the user over time; and estimating the position of the handheld personal care device relative to the user using the obtained measurements and based on a predetermined relationship between the weight distribution and/or the torque imparted and the position of the handheld personal care device relative to the user.

## Description

### TECHNICAL FIELD OF THE INVENTION

The invention relates to an apparatus, and techniques for estimating a position of a handheld personal care device, such as a shaver or toothbrush, relative to a user of the handheld personal care device.

### BACKGROUND TO THE INVENTION

It is envisaged that handheld personal care devices, such as electric shavers, razors, toothbrushes (electric and non-electric), air floss devices, skin treatment applicators, facial cleansers, eye energisers, epilators, etc., will start to offer a more meaningful interaction between the device and a user of the device. The prevalence of "connected products" (e.g. products that are connected to the Internet or a home network) is increasing and such products have sensing functionality to generate data about the use or operation of the products. The functionality of handheld personal care devices could be improved by exploiting the data that has become available: solutions can be personalised, tuned to the moment and will create improved primary performance and experience.

An important aspect of providing personalised information and tuning to the moment is the position of the device with respect to the body of the user (i.e. the person that the device is being used on). This localization information could allow the device to automatically adjust its settings to the actual location of the device in order to optimise the performance (for example different treatment areas have different characteristics and thus different treatment demands). Furthermore, localization information could provide other valuable information to the user, such as usage logging, overtreatment warning, advice (e.g. the setting that is best to be used based on results of previous treatments), feedback (e.g. progress of treatment results) and compliance to a treatment procedure or treatment program. Finally, such information could also be sent through the Internet and made available to the manufacturer of the device. Combining information from a multitude of users can provide new insights into the use of devices by consumers.

Handheld personal care devices are controlled by a user and conventionally they do not comprise or use any sensor to detect their location and orientation of use. Having the ability to detect the orientation of the device during use and/or the position of the device relative to a user (e.g. where on the body of the user the device is being used) is useful, and in some cases could be used to adapt one or more operating parameters of the device to the benefit of the user.

For example, in the case of facial cleansers with a rotating brush, the brush will typically rotate in one direction which does not provide the optimal treatment to all locations on the face. Instead, it can be useful for the direction of rotation of the brush to depend on whether the device is being used on the left or right side of the face. Currently this change in direction can be achieved manually by the user by pressing a button or based on a specific treatment time after which the direction of the brush is automatically changed. However, an improved automated system would be useful.

Current systems for determining the position of a device can make use of accelerometers and/or gyroscopes or another form of inertial measurement unit (IMU) to determine the absolute orientation of the device in relation to gravity, and relative orientation on other axes. However, these systems suffer from accumulated error or drift over time and require regular recalibration. Alternatively, systems can use a locally-generated static magnetic field to determine the exact position and orientation of the device in relation to the user's head. However, the user needs to wear ear plugs or another type of head-mounted device to generate the magnetic field (or sense the magnetic field if it is generated by the device) in order for the device to sense its position and orientation in relation to the ear plugs. In yet another alternative, a remote camera can be used to monitor the user, and image processing techniques can be used to identify the user, the device and their relative positions. However, this solution creates privacy concerns, particularly if the camera is located in a bathroom, and requires complex image processing techniques. Another solution is to use a body map as a reference, e.g. using skin features. However, the reference map has to be created per user before use of the device, and significant processing power and data storage is required.

Thus, it is difficult to determine the position of a personal care device since such a device is a dynamic object and the body (including the head) of the user is also a dynamic object, and there is therefore a need for an improved technique for estimating the position of a handheld personal device relative to a user of the handheld personal care device.

### SUMMARY OF THE INVENTION

It has been appreciated that, when a user uses a handheld personal care device on the face or in the neck area, the user (whether consciously or unconsciously) turns his or her head in order to properly and easily use the device. This is particularly the case where the body part being treated is in front of a mirror, so that the user can better view the treatment area (i.e. the area or part of the body that the handheld personal care device is being used on).

Turning the head provides the user with a better view of the treatment area in a mirror, and means that the user does not have to displace/reposition his or her hand or arm into a less convenient position during the treatment. For example, when using a shaver, a user typically turns the head opposite to where the device is to be positioned - a user turns the head to the left when he wants to shave the right cheek, or the user raises the head to shave the neck area.

A result of this rotation or movement of the head is that the overall posture of the user changes, for example causing a small rotation within the full body (torque) while keeping the feet in the same position on the ground.

It has also been found that when using a device in front of the mirror the user may bend slightly towards the mirror (in order to get a better view of the treatment area) as well as turn or move the head, and this results in a repositioning of the body weight to a certain standing leg while the feet remain in the same position on the ground.

Thus, based on the above findings, it is proposed to estimate a position of a handheld personal care device relative to a user of the handheld personal care device based on measurements of a weight distribution of the user over time in a predetermined area (e.g. a part of a floor or a weighing scales) and/or based on measurements of a torque imparted to the predetermined area over time by one or both feet of the user, and a predetermined relationship between the weight distribution and/or the torque imparted and the position of the handheld personal care device relative to the user.

Therefore, according to a first aspect of the invention, there is provided a method of estimating a position of a handheld personal care device relative to a user of the personal care device, the method comprising obtaining one or both of (i) measurements of a weight distribution of the user in a predetermined area over time and (ii) measurements of a torque imparted to the predetermined area by one or both feet of the user over time; and estimating the position of the handheld personal care device relative to the user using the obtained measurements and based on a predetermined relationship between the weight distribution and/or the torque imparted and the position of the handheld personal care device relative to the user.

In some embodiments, the predetermined relationship defines a respective characteristic of the weight distribution and/or the torque imparted for each of a plurality of positions of the handheld personal care device relative to the user.

In some embodiments, the predetermined relationship defines at least a first weight distribution and/or a first torque imparted for a first position of the handheld personal care device relative to the user and a second weight distribution and/or a second torque imparted for a second position of the handheld personal care device relative to the user.

In these embodiments, the first position maybe a position on the left side of the user, and the second position may be a position on the right side of the user.

In some embodiments, the measurements of the weight distribution of the user in the predetermined area over time indicate the position of a centre of gravity of the user in the predetermined area over time.

In some embodiments, the position of the handheld personal care device relative to the user is a position of the handheld personal care device relative to the head of the user.

In these embodiments, the predetermined relationship may define that the handheld personal care device is on the left side of the head of the user if the measured weight distribution indicates that a centre of gravity of the weight distribution is in a right half of the predetermined area, and that the handheld personal care device is on the right side of the head of the user if the measured weight distribution indicates that the centre of gravity of the weight distribution is in a left half of the predetermined area.

In these embodiments, the predetermined relationship may define that the handheld personal care device is on the front side of the head of the user if the measured weight distribution indicates that a centre of gravity of the weight distribution is in or near the middle of the predetermined area.

In these embodiments, the predetermined relationship may define that the handheld personal care device is on or near the neck of the user if the measured weight distribution indicates that a centre of gravity of the weight distribution is in a rear half of the predetermined area.

In alternative embodiments, the predetermined relationship defines that the handheld personal care device is on the left side of the head of the user if the measured torque imparted indicates that the user is turned to the right, and that the handheld personal care device is on the right side of the head of the user if the measured torque imparted indicates that the user is turned to the left.

In these embodiments, the predetermined relationship may define that the handheld personal care device is on the front side of the head of the user if the measured torque imparted indicates that the user is facing forwards.

In some embodiments, the method further comprises the step of estimating a motion of the handheld personal care device using the obtained measurements.

In these embodiments, the step of estimating a motion of the handheld personal care device may comprise estimating a direction of an oscillating motion of the handheld personal care device.

In these embodiments, the step of estimating a motion of the handheld personal care device may comprise estimating the motion from changes in the weight distribution of the user and/or changes in the measured torque imparted over time.

According to a second aspect of the invention, there is provided a computer program product comprising a computer readable medium having computer readable code embodied therein, the computer readable code being configured such that, on execution by a suitable computer or processor, the computer or processor is caused to perform any of the methods according to the first aspect of the invention described above.

According to a third aspect of the invention, there is provided an apparatus for estimating a position of a handheld personal care device relative to a user of the handheld personal care device, the apparatus comprising a control unit that is configured to obtain one or both of (i) measurements of a weight distribution of the user in a predetermined area over time and (ii) measurements of a torque imparted to the predetermined area by one or both feet of the user over time; and estimate the position of the handheld personal care device relative to the user using the obtained measurements and based on a predetermined relationship between the weight distribution and/or the torque imparted and the position of the handheld personal care device relative to the user.

In some embodiments, the predetermined relationship defines a respective characteristic of the weight distribution and/or the torque imparted for each of a plurality of positions of the handheld personal care device relative to the user.

In some embodiments, the predetermined relationship defines at least a first weight distribution and/or a first torque imparted for a first position of the handheld personal care device relative to the user and a second weight distribution and/or a second torque imparted for a second position of the handheld personal care device relative to the user.

In these embodiments, the first position maybe a position on the left side of the user, and the second position can be a position on the right side of the user.

In some embodiments, the measurements of the weight distribution of the user in the predetermined area over time indicate the position of a centre of gravity of the user in the predetermined area over time.

In some embodiments, the position of the handheld personal care device relative to the user is a position of the handheld personal care device relative to the head of the user.

In these embodiments, the predetermined relationship may define that the handheld personal care device is on the left side of the head of the user if the measured weight distribution indicates that a centre of gravity of the weight distribution is in a right half of the predetermined area, and that the handheld personal care device is on the right side of the head of the user if the measured weight distribution indicates that a centre of gravity of the weight distribution is in a left half of the predetermined area.

In these embodiments, the predetermined relationship may define that the handheld personal care device is on the front side of the head of the user if the measured weight distribution indicates that a centre of gravity of the weight distribution is in or near a middle of the predetermined area.

In these embodiments, the predetermined relationship may define that the handheld personal care device is on or near the neck of the user if the measured weight distribution indicates that a centre of gravity of the weight distribution is in a rear half of the predetermined area.

In alternative embodiments, the predetermined relationship defines that the handheld personal care device is on the left side of the head of the user if the measured torque imparted indicates that the user is turned to the right, and that the handheld personal care device is on the right side of the head of the user if the measured torque imparted indicates that the user is turned to the left.

In these embodiments, the predetermined relationship may define that the handheld personal care device is on the front side of the head of the user if the measured torque imparted indicates that the user is facing forwards.

In some embodiments, the control unit is further configured to estimate a motion of the handheld personal care device using the obtained measurements.

In some embodiments, the control unit is configured to estimate the motion of the handheld personal care device by estimating a direction of an oscillating motion of the handheld personal care device.

In some embodiments, the control unit is configured to estimate the motion of the handheld personal care device by estimating the motion from changes in the weight distribution of the user and/or changes in the measured torque imparted over time.

In some embodiments, the apparatus further comprises one or both of a weight sensor for measuring the weight distribution of the user in the predetermined area, and a torque sensor for measuring the torque imparted to the predetermined area by one or both feet of the user. In other embodiments, the control unit is configured to be connected to one or both of a weight sensor for measuring the weight distribution of the user in the predetermined area, and a torque sensor for measuring the torque imparted to the predetermined area by one or both feet of the user.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates a user using a shaver to shave four different parts of his head from the point of view of a mirror in front of the user;
Figure 2 is an illustration of the parameterisation of head movements into three directions of rotation;
Figure 3 is a block diagram of an apparatus according to the invention;
Figure 4 shows an exemplary weight distribution measurement apparatus;
Figure 5 shows an exemplary torque measurement apparatus;
Figure 6 is a flow chart that illustrates a method of estimating a position of a handheld personal care device relative to a user according to the invention;
Figure 7 illustrates an exemplary mapping or predetermined relationship between the weight distribution of a user and the position of a handheld personal care device relative to the user;
Figure 8 shows three graphs illustrating measurements of the weight distribution of the user for three different users performing a personal care treatment using a handheld personal care device;
Figure 9 is a set of graphs illustrating the identification of a direction of movement of a handheld personal care device; and
Figure 10 illustrates an exemplary mapping or predetermined relationship between the torque imparted by the feet of the user and the position of a handheld personal care device relative to a user.

### DETAILED DESCRIPTION OF THE PREFERRED EMBODIMENTS

The position of a handheld personal care device, such as a shaver or toothbrush, changes as the handheld personal care device is used by the user. The posture of the user can also change as the handheld personal care device is used by the user. In particular, when using a handheld personal care device in front a mirror, a user may turn his or her head to provide a better view of the treatment area. For example, when using a shaver, a user typically turns the head opposite to where the device is to be positioned, e.g. a user turns the head to the left when he wants to shave the right cheek, or the user raises the head to shave the neck area, etc. When the user is to treat a different area (e.g. the left cheek), he will change his posture to provide a better view in the mirror of that treatment area (e.g. he will turn the head to the right in order to shave the left cheek).

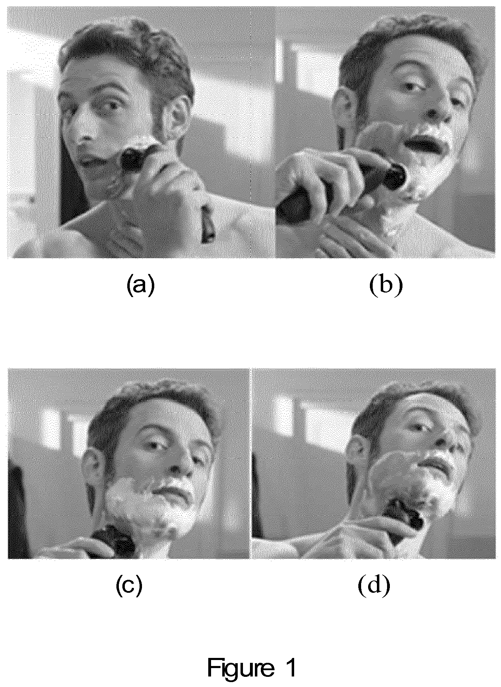

Figure 1 illustrates a user using a shaver to shave four different parts of the head from the point of view of a mirror in front of the user, and shows the typical head postures that a user adopts when shaving those body parts. In Figure 1(a) the user is shaving the left cheek and the head is turned to the right. In Figure 1(b) the user is shaving the right side of the chin and the head is turned to the left. In Figure 1(c) the user is shaving the right hand side of the neck and the head is turned to the left and slightly upwards. In Figure 1(d) the user is shaving under the right side of the chin and the head is turned upwards and slightly to the right.

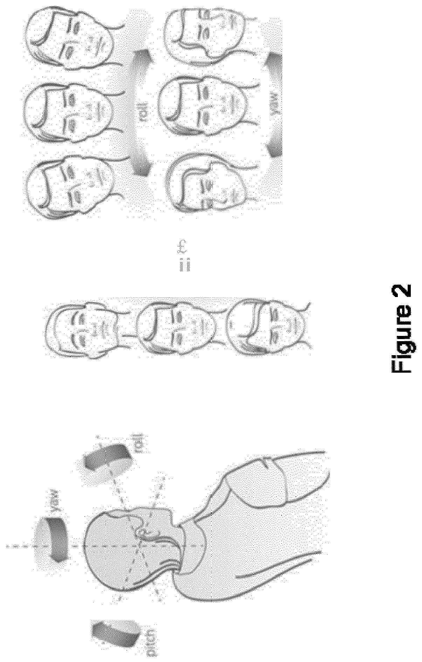

Figure 2 illustrates that head movements can be uniquely described using only three parameters: pitch, yaw and roll. Thus, pitch relates to rotation in a generally forward and back direction (e.g. nodding), yaw relates to left or right rotation around an axis parallel to the neck, and roll relates to the tilting of the head to the left or right.

The posture change described above as the handheld personal care device is used on different parts of the body of the user can result in a change of the weight distribution of the user over a floor region. It has also been found that moving the device to different parts of the body of the user, without the posture of the user changing, can also lead to a change of the weight distribution of the user over a floor region due to the weight of the device being repositioned with respect to the body of the user.

Furthermore, the posture change of the user, or turning of one body part (e.g. the head) relative to another (e.g. the chest) can result in a torque in the body of the user, and this torque can be imparted to the floor via the user's foot or feet.

Thus, the techniques described herein estimate the position of a handheld personal care device relative to a user based on measurements of the distribution of the weight of the user over time in a predetermined area (e.g. a part of the floor or weighing scales) and/or based on measurements of the torque imparted to a predetermined area (e.g. a part of the floor or weighing scale) over time by one or both feet of the user, and based on a predetermined relationship between the weight distribution and/or the torque imparted and the position of the handheld personal care device relative to the user.

The term "user" is used throughout this document to refer to the person on which the handheld personal care device is being used. For example the user is the person whose teeth are being brushed or whose face is being shaved. The "user" is also the person to which the relative position of the handheld personal care device is estimated. The term "user" does not necessarily refer to the person that is operating or controlling the handheld personal care device. For example the handheld personal care device may be operated by another person or operator. In the embodiments described below, it is assumed that the user is using the handheld personal care device on himself, but it should be appreciated that the embodiments are also applicable to situations where the handheld personal care device is operated by another person or operator to perform the personal care activity on the user.

As noted above, the invention is for using a handheld personal care device for performing a personal care activity on or to a user. The handheld personal care device can be for use on a specific part of the body, e.g. the teeth or the face, or it could be for use generally on the body, e.g. it could be for shaving or cutting hair on the face, legs, arms, etc.

The handheld personal care device can be any type of device that is held in the hand and that is for performing any type of personal care activity. For example, and without limitation, the handheld personal care device can be any of electric shaver, a handheld razor (i.e. without any moving or actuated parts), hair clippers, a beard trimmer, a manual toothbrush (i.e. without any moving or actuated parts), an electric toothbrush, an 'air floss' device, a skin massager, a skin treatment applicator, a facial cleanser, an eye energiser, an epilator, etc.

The handheld personal care device will include a personal care component that is for performing a personal care activity on a user, or is for use in performing a personal care activity on a user. The nature or type of personal care component depends on the nature or type of the handheld personal care device. For example where the handheld personal care device is an electric shaver, the personal care component can comprise a shaving head, including one or more blades or other cutting element(s), and an actuator (for example a motor) for actuating the cutting element to create a cutting action. In another example, where the handheld personal care device is an electric toothbrush, the personal care component can comprise a brush head and an actuator for actuating the brush head (e.g. rotating, oscillating and/or vibrating the brush head) to create a brushing action. In yet another example, the handheld personal care device is a manual toothbrush, and the personal care component is a static brush head. It will be appreciated from the following description of the invention that the invention can be implemented or used with any type of handheld personal care device and thus any type of personal care component, and the examples provided above should not be considered to be limiting.

It will be appreciated that in some embodiments the personal care component can comprise one or more elements, such as an actuator, whose operating parameter(s) can vary or be varied during use of the handheld personal care device. For example a direction and/or speed of rotation of an actuator can be changed during use, or a cutting length of a cutting element can be changed during use, etc. In these embodiments, the personal care component can receive a control signal or control signals that set or control the operating parameter(s) of the personal care component.

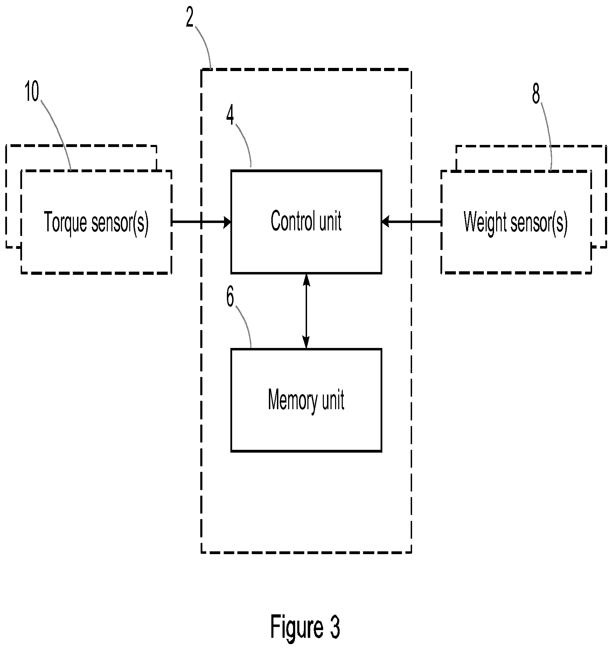

Figure 3 is a block diagram of an apparatus 2 for estimating a position of a handheld personal care device with respect to a user according to the invention. In some embodiments the apparatus 2 is part of a handheld personal care device (which is also referred to herein as a 'device'). However, in alternative embodiments the apparatus 2 is separate from the handheld personal care device, and the handheld personal care device can be conventional.

The apparatus 2 comprises a control unit 4 that is configured to perform the method according to the invention to estimate the position of the handheld personal care device relative to the user, and, in some embodiments, to control the operation of a personal care component in the handheld personal care device, or more generally control the operation of the handheld personal care device, based on the estimated position of the handheld personal care device by sending one or more control signals to the personal care component.

As noted above, in some embodiments the control unit 4 is part of the same unit as the personal care component/handheld personal care device, but in other embodiments the control unit 4 and/or the apparatus 2 is separate from the handheld personal care device. For example in some embodiments the apparatus 2 can be a base unit for the handheld personal care device, e.g. a docking and/or charging station, but in other embodiments (since the apparatus 2 does not require direct interaction with the handheld personal care device in order to estimate the position of the handheld personal care device) the apparatus 2 can be completely independent of the handheld personal care device. For example the apparatus 2 can be implemented as a computer or other electronic device, such as a laptop, tablet or smartphone. In some embodiments, the apparatus 2 can be part of a so-called 'virtual' mirror (which comprises a display panel and a camera that records the user and displays the recorded (live) images on the display panel), or part of a 'smart bathroom' control hub (e.g. a device that controls one or more connected devices or components used in a bathroom environment).

The control unit 4 can be implemented in numerous ways, with software and/or hardware, to perform the various functions described below. The control unit 4 may comprise one or more microprocessors or digital signal processors (DSPs) that may be programmed using software or computer program code to perform the required functions and/or to control components of the control unit 4 to effect the required functions. The control unit 4 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

In various implementations, the control unit 4 may be associated with or comprise one or more memory units 6 such as volatile and non-volatile computer memory such as RAM, PROM, EPROM, and EEPROM. The control unit 4 or associated memory unit 6 can also be used for storing program code that can be executed by a processor in the control unit 4 to perform the method described herein.

As noted above, the position of the handheld personal care device with respect to the user is estimated according to the invention using measurements of the weight distribution of the user over time in a predetermined area and/or based on measurements of the torque imparted to the predetermined area over time by one or both feet of the user. Therefore the control unit 4 is configured to receive measurements of the weight distribution in the predetermined area over time and/or to receive measurements of the torque imparted to the predetermined area by one or both feet of the user. These measurements can be provided by one or more weight sensors 8 and one or more torque sensors 10 respectively.

In some embodiments, the one or more weight sensors 8 and one or more torque sensors 10 can be part of the apparatus 2, but in other embodiments the one or more weight sensors 8 and one or more torque sensors 10 can be separate from the apparatus 2. In the latter case the control unit 4 and/or apparatus 2 can be connected to the sensors 8, 10 to receive measurement signals from the sensors 8, 10. The connection can be a wired or wireless connection. In the case of a wireless connection, the apparatus 2 may further comprise suitable components (e.g. transceiver circuitry) that can be used to receive the measurement signal(s) from the sensors 8, 10.

The one or more weight sensors 8 can comprise any type of sensor that can be used to measure the weight or force applied to an object. For example the weight sensor 8 could be a strain gauge, a load cell, a piezoelectric crystal, a force sensor, a pressure sensor, etc. Each weight sensor 8 measures the force or weight exerted and outputs a respective measurement signal. In some embodiments, a single weight sensor 8 can provide a measurement of the weight distribution in a particular area (and thus a change in the weight distribution over time can be determined), but in other embodiments, a plurality of weight sensors 8 can be distributed over a particular area, with each weight sensor 8 providing a respective measurement signal for a part of that area.

By analysing the measurement signals from the single weight sensor 8, or from each of the plurality of weight sensors 8, the control unit 4 can determine the weight distribution in an area associated with the weight sensor(s) 8 (this area is referred to as a 'predetermined area' herein). In particular, the control unit 4 can determine where in the predetermined area the centre of gravity of the weight distribution or the user is located. The control unit 4 can analyse measurement signal(s) received from the weight sensor(s) 8 over time to determine changes in the weight distribution (e.g. changes in the position of the centre of gravity of the weight distribution or the user) over time.

In some embodiments, the weight sensor(s) 8 are integrated into the floor in the environment in which a user typically uses a handheld personal care device. For example the weight sensor(s) 8 can be part of a floor tile or floor tiles, and can be located in an area where a user typically sits or stands when using a handheld personal care device. In other embodiments, the weight sensor(s) 8 are part of an object that can be moved/removed/placed in an area in which a user typically sits or stands when using a handheld personal care device. For example the object could be a floor mat or a set of weighing scales that already comprises one or more weight sensor(s) 8.

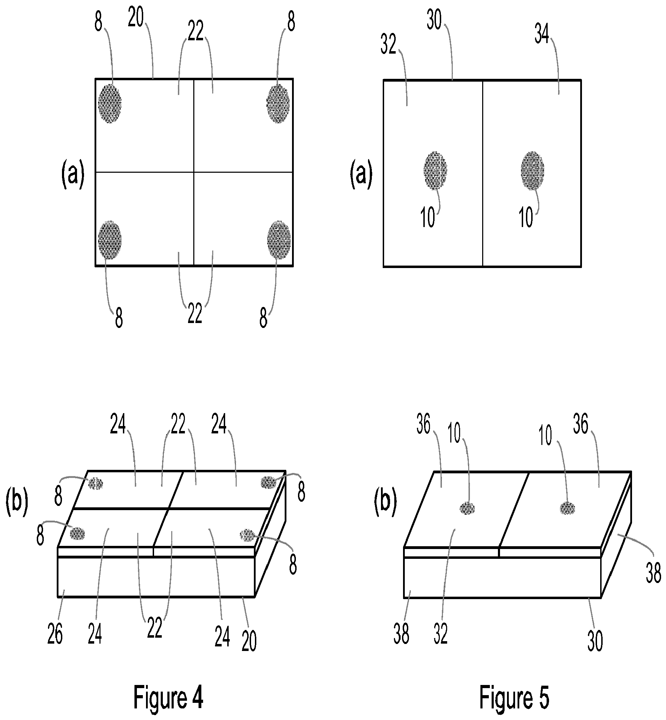

Figure 4 shows an exemplary weight distribution measurement apparatus 20 that can be used to measure the weight distribution in a particular area. The weight distribution measurement apparatus 20 shown in Figure 4 is generally rectangular (although it will be appreciated that the weight distribution measurement apparatus 20 can take any desired shape) and comprises four weight sensors 8, each positioned in a respective quadrant 22 of the weight distribution measurement apparatus 20.

Figure 4(a) shows a top view of the weight distribution measurement apparatus 20, and it can be seen that each weight sensor 8 is located towards the outer corner of each quadrant 22. However, it will be appreciated that the illustrated positions of the weight sensors 8 is not limiting. Figure 4(b) is a perspective view of the weight distribution measurement apparatus 20, and shows that each weight sensor 8 can be positioned between a surface portion 24 that the user directly stands or sits on and a base portion 26. The weight distribution measurement apparatus 20 can take any desired form. For example the weight distribution measurement apparatus 20 can be in the form of a floor tile, a floor mat, a pressure pad, weighing scales, etc. Although not shown in Figure 4, the weight distribution measurement apparatus 20 can comprise suitable components and/or circuitry for providing the measurement signal(s) to the control unit 4. For example the weight distribution measurement apparatus 20 can include a wired connection or transmitter/transceiver circuitry for communicating the measurement signal(s) to the control unit 4.

It will be appreciated that weight distribution measurement apparatus are known in the art. The Wii^{™} Balance Board is an example of a weight distribution measurement apparatus that could be used to obtain weight distribution/centre of gravity measurements for use in the invention.

The one or more torque sensor(s) 10 can comprise any type of sensor that can be used to measure the torque imparted on an object. For example the torque sensor 10 can be a strain gauge. Each torque sensor(s) 10 measures the torque imparted and outputs a respective measurement signal. In some embodiments, a single torque sensor 10 maybe used to provide a measurement of the torque imparted in a particular area (also referred to as a 'predetermined area' herein), but in other embodiments, a plurality of torque sensors 10 can be distributed over a particular area, with each torque sensor 10 providing a respective measurement signal indicative of the torque imparted on a respective part of the particular area. For example, two torque sensors 10 can be used, with each torque sensor 10 being used to measure the torque imparted by a respective foot of the user.

In some embodiments, each torque sensor 10 measures the torque around a single axis, for example a vertical axis (so as to measure torque in a horizontal plane) or a horizontal axis. In other embodiments, each torque sensor 10 can measure the torque around multiple axes, for example a vertical axis and/or one or more horizontal axes.

The control unit 4 can analyse measurement signal(s) received from the torque sensor(s) 10 over time to determine changes in the torque imparted over time.

In some embodiments, the torque sensor(s) 10 are integrated into the floor in the environment in which a user typically uses a handheld personal care device. For example the torque sensor(s) 10 can be part of a floor tile or floor tiles, and can be located in an area where a user typically sits or stands when using a handheld personal care device. In other embodiments, the torque sensor(s) 10 are part of an object that can be moved/removed/placed in an area in which a user typically sits or stands when using a handheld personal care device. For example the object could be a floor mat or a set of weighing scales.

Figure 5 shows an exemplary torque measurement apparatus 30 that can be used to measure the torque imparted in a particular area. The torque measurement apparatus 30 shown in Figure 5 is generally rectangular (although it will be appreciated that the torque measurement apparatus 30 can take any desired shape) and comprises two torque sensors 10, each positioned in a respective half portion 32, 34 of the torque measurement apparatus 30. The left half portion 32 is provided to measure the torque imparted by the left foot of a user and the right half portion 34 is provided to measure the torque imparted by the right foot of a user.

Figure 5(a) shows a top view of the torque measurement apparatus 30, and it can be seen that each torque sensor 10 is located in the centre of each half portion 32, 34. However, it will be appreciated that the illustrated positions of the torque sensors 10 is not limiting. Figure 5(b) is a perspective view of the torque measurement apparatus 30, and shows that each torque sensor 10 can be positioned between a surface portion 36 that the user directly stands or sits on and a base portion 38. As with the weight distribution measurement apparatus 20, the torque measurement apparatus 30 can take any desired form. For example the torque measurement apparatus 30 can be in the form of a floor tile, a floor mat, a pressure pad, weighing scales, etc. Although not shown in Figure 5, the torque measurement apparatus 30 can comprise suitable components and/or circuitry for providing the measurement signal(s) to the control unit 4. For example the torque measurement apparatus 30 can include a wired connection or transmitter/transceiver circuitry for communicating the measurement signal(s) to the control unit 4.

It will be appreciated that in some embodiments the control unit 4 can use measurements of both the weight distribution and the torque imparted to estimate the position of the handheld personal care device relative to the user. In that case, a measurement device can be provided that includes both weight sensor(s) 8 and torque sensor(s) 10. For example, a measurement device can be provided that corresponds to a combination of the measurement devices 20, 30 shown in Figures 4 and 5.

It will be appreciated that the components and features of the apparatus 2 shown in Figure 3 are not exhaustive, and an actual implementation of an apparatus 2 will include further components and features to those shown and described above. For example the apparatus 2 may include a power supply, such as a battery, or means for connecting the apparatus 2 to a power supply. Alternatively or in addition, the apparatus 2 may comprise one or more buttons, controls, or other user interface elements to allow a user to control the operation of the apparatus 2. In addition, the apparatus 2 may include transceiver circuitry and/or other communication means to enable the apparatus 2 to communicate with other components (for example the weight sensor(s) 8 and torque sensor(s) 10). The transceiver circuitry can be any suitable wireless module, including, for example, radio or infra-red transmitters and receivers. Suitable wireless technologies include Bluetooth, Zigbee, Wi-Fi, etc. Transceiver circuitry can also or alternatively be provided where the apparatus 2 is to provide information on the usage and/or operation of the apparatus 2 or handheld personal care device to another system, for example operated by the manufacturer of the apparatus 2, so that the manufacturer can monitor how consumers are using their handheld personal care devices, and/or to another electronic device belonging to the user or operator of the device (such as a computer, smart phone, tablet, etc.) so that the user or operator can review and/or monitor the usage of the handheld personal care device.

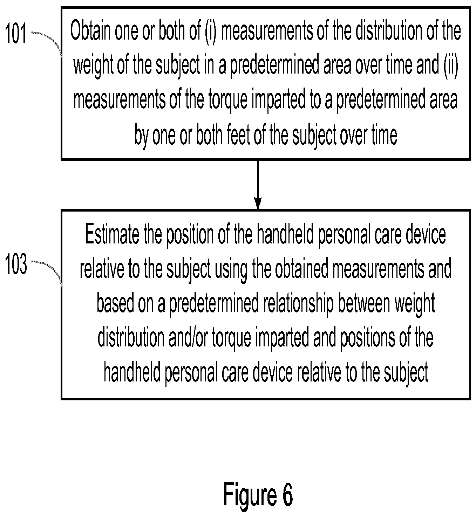

Figure 6 is a flow chart that illustrates a method of estimating the position of a handheld personal care device relative to a user according to the invention. It will be appreciated that the method in Figure 6 can be performed by the apparatus 2 and/or the control unit 4. In some embodiments, the control unit 4 can perform the method in response to executing suitable software or computer program code, for example that is stored in the memory unit 6.

Thus, in a first step, step 101, measurements are obtained. The measurements can be measurements of the weight distribution of the user in a predetermined area over time, and/or measurements of the torque imparted to a predetermined area by one or both feet of the user over time. Obtaining the measurements can mean that the control unit 4 receives measurement signals from one or more weight sensor(s) 8 and/or one or more torque sensor(s) 10, or measuring the weight distribution and/or the imparted torque and providing the measurements to the control unit 4.

Next, in step 103, the position of the handheld personal care device relative to the user is estimated using the obtained measurements and a predetermined relationship between the weight distribution and/or the torque imparted and the position of the handheld personal care device relative to the user.

In some embodiments, the predetermined relationship can map a particular weight distribution (e.g. centre of gravity position in the predetermined area), or range of weight distributions (e.g. in terms of a part of the predetermined area in which the centre of gravity can be located) to a particular position of the handheld personal care device relative to the user. Likewise, the predetermined relationship can map a particular imparted torque (or torque pattern in the case where multiple torque sensors 10 are used), or range of torques to a particular position of the handheld personal care device relative to the user.

It will be appreciated that the position of the handheld personal care device relative to the user may be estimated with varying degrees of resolution. For example, in some embodiments, the position can be determined in terms of which side of the body the device is on (e.g. left or right, or front or back). In other embodiments, the position can be determined in terms of which body part the device is on or near (e.g. on the head, neck, or chest).

In some embodiments, the predetermined relationship is based on the typical behaviour (i.e. in terms of weight distribution and/or torque imparted) of a user when a handheld personal care device is being used in a particular position relative to the user. For example the predetermined relationship can be based on an analysis and average of measured behaviours for a population of users. In other embodiments, the predetermined relationship is determined from an analysis of the behaviour of the particular user that is using the handheld personal care device, e.g. as determined during a calibration phase when the handheld personal care device is first used by the user. In a calibration procedure, the handheld personal care device can be moved to one or more predetermined positions with respect to the user and measurements of the weight distribution and/or the imparted torque obtained for each of those positions. A mapping between weight distribution and/or the imparted torque for each of these positions can be determined from an analysis of the measurements.

In some embodiments, step 103 can comprise analysing the obtained measurements to determine one or more parameters and/or identify one or more events. In particular, the measurements could be analysed to determine any of: the centre of gravity and/or the centre of balance of the user, a change in weight distribution and/or location of centre of gravity, for example from the user changing from favouring one leg to another; a change in torque imparted.

In some embodiments, the obtained measurements can be analysed in the frequency domain to detect an oscillating movement of the handheld personal care device by the user (for example moving the device from side to side, e.g. in the case of a toothbrush) or in a circular motion, e.g. in the case of a shaver). In some embodiments, information on the direction of an oscillating movement can be used as an indirect indication of the position of the device. For example, movement in certain directions could be associated with a particular position of the device relative to the user (or not associated with a particular position). For example a left-right movement might be associated with the device being positioned at or on the forehead, whereas a forward-back and/or up-down movement might be associated with the cheek). In addition or alternatively, the obtained measurements can be analysed in the frequency domain to identify unique device-related vibrational frequencies to enable identification of the handheld personal care device.

In some embodiments, the predetermined relationship used in step 103 defines a respective characteristic of the weight distribution and/or the torque imparted for each of a plurality of positions of the handheld personal care device relative to the user. That is, the predetermined relationship can define at least a first weight distribution and/or a first torque imparted for a first position of the handheld personal care device relative to the user, and a second weight distribution and/or a second torque imparted for a second position of the handheld personal care device relative to the user. In some embodiments, the first position is a position on the left side of the user and the second position is a position on the right side of the user.

In some embodiments, the position of the handheld personal care device relative to the user is a position of the handheld personal care device relative to the head of the user.

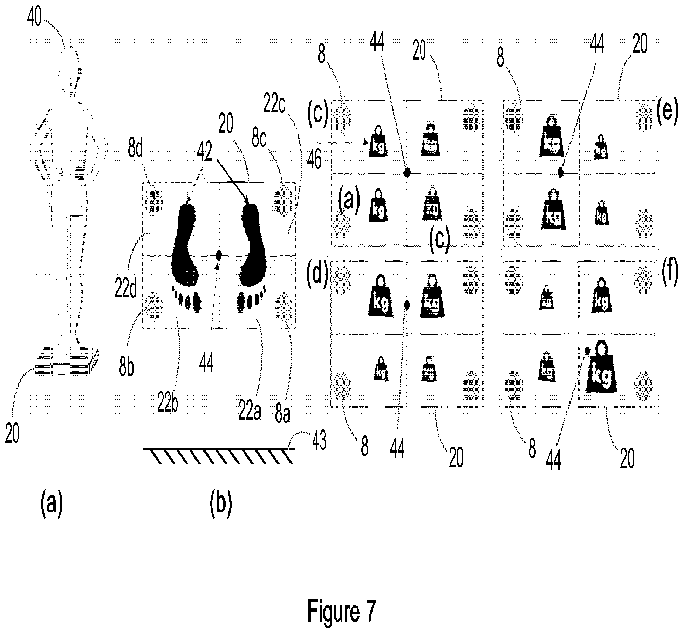

Figure 7 illustrates an exemplary mapping or predetermined relationship between the weight distribution and the position of a handheld personal care device relative to a user.

Figure 7(a) shows a user 40 standing on a weight distribution measurement apparatus 20 as described above with reference to Figure 4. Figure 7(b) shows a top view of the weight distribution measurement apparatus 20 with the feet 42 of the user 40 shown positioned on the weight distribution measurement apparatus 20. The user 40 is standing on the weight distribution measurement apparatus 20 in front of a mirror 43. As in Figure 4, the weight distribution measurement apparatus 20 comprises four quadrants 22a-d, each with a corresponding weight sensor 8a-d. Quadrant 22a is referred to as the front left quadrant, quadrant 22b is referred to as the front right quadrant, quadrant 22c is referred to as the back left quadrant, and quadrant 22d is referred to as the back right quadrant. It will be appreciated that quadrants 22 have been labelled from the point of view of the user 40. Thus, in Figure 7(b), the left foot 42 of the user 40 is positioned across the front left quadrant 22a and back left quadrant 22c, and the right foot 42 of the user 40 is positioned across the front right quadrant 22b and back right quadrant 22d.

When the user 40 is standing upright directly facing the mirror 43, the weight of the user (and handheld personal care device if the user is holding the device) is distributed evenly amongst the quadrants 22, and thus the centre of gravity 44 of the user 40 is shown in Figure 7(b) in the middle of the four quadrants 22.

This scenario is also shown in Figure 7(c), where the weight measured in each quadrant is represented by a respective weight symbol 46, where the relative sizes of the weight symbols 46 in each of the quadrants 22 indicates the relative distribution of the weight. Thus, in Figure 7(c), the user 40 is standing upright facing the mirror 43 and the weight is evenly distributed across the four quadrants 22, with the centre of gravity of the weight distribution in the middle of the four quadrants 22.

In Figure 7(d), the user 40 is using a handheld personal care device on the neck and has tilted the head back. This has shifted some of the weight of the user 40 backwards (i.e. away from the mirror 43), so that there is proportionally more weight in the back left quadrant 22c and back right quadrant 22d, and proportionally less weight in the front left quadrant 22a and front right quadrant 22b. The centre of gravity 44 of the weight distribution has therefore shifted away from the mirror 43 and is between the back left quadrant 22c and back right quadrant 22d.

In Figure 7(e), the user 40 is using a handheld personal care device on the left side of the head and has turned the head to the right and leant to the right to better see that area in the mirror 43. This has shifted some of the weight of the user 40 to the right, so that there is proportionally more weight in the front right quadrant 22b and back right quadrant 22d, and proportionally less weight in the front left quadrant 22a and back left quadrant 22c. The centre of gravity 44 of the weight distribution has therefore shifted to the right from centre and is between the front right quadrant 22b and the back right quadrant 22d.

In Figure 7(f), the user 40 is using a handheld personal care device on the front right side of the head and has turned the head to the left and leant forward to better see that area in the mirror 43. This has shifted some of the weight of the user 40 to the front left, so that there is proportionally more weight in the front left quadrant 22a, with proportionally less weight in the front right quadrant 22b, back left quadrant 22c and back right quadrant 22d. The centre of gravity 44 of the weight distribution has therefore shifted into the front left quadrant 22a.

Thus, based on the above, in some embodiments, the predetermined relationship defines that the handheld personal care device is on the left side of the head of the user 40 if the measured weight distribution indicates that the centre of gravity of the weight distribution is in a right half of the predetermined area (as measured from the point of view of the user 40), and that the handheld personal care device is on the right side of the head of the user 40 if the measured weight distribution indicates that the centre of gravity of the weight distribution is in a left half of the predetermined area.

In some embodiments, the predetermined relationship defines that the handheld personal care device is on the front side of the head of the user 40 (from the point of view of the user 40) if the measured weight distribution indicates that the centre of gravity of the weight distribution is in or near the middle of the predetermined area.

In some embodiments, the predetermined relationship defines that the handheld personal care device is on or near the neck of the user 40 if the measured weight distribution indicates that the centre of gravity of the weight distribution is in the rear half of the predetermined area.

It has been found that the following actions can be determined from measurements of the weight distribution in a predetermined area.

**Table 1**

| **Action** | **Measurement** |
|---|---|
| Standing on the floor sensor unit | Initial weight of person |
| Grabbing device | Leaning forward and adding weight to weight distribution measurement apparatus 20 (inverse of Figure 7(d)). Additional weight due to the device being picked up could potentially be used to identify and characterise the type of device. |
| Using device on right/left area | Detection of weight redistribution (e.g. as in Figure 7(f)) to right/left and front |
| Up and down movement of device | Oscillation in total force/weight across all weight sensors 8 due to device oscillating movement |
| Left and right movement of device | Oscillation in force/weight distribution to left and right side |
| Front and back movement of device | Oscillation in force/weight distribution from front to back side |
| More complex movement patterns of device | Combination of the above three examples. |

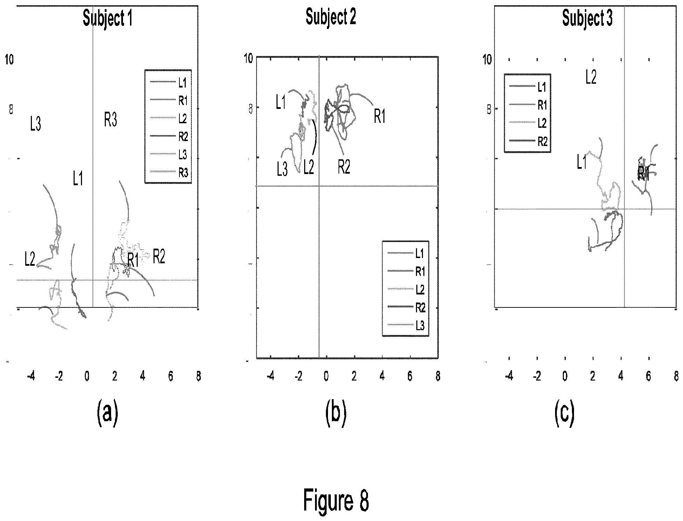

Figure 8 shows three graphs illustrating measurements of the weight distribution for three different users performing a personal care treatment using a handheld personal care device. The area of each graph represents the predetermined area, and the lines in the graph represent the changes in the weight distribution (specifically the centre of gravity of the weight distribution) within the predetermined area during treatment of different sides of the user's face. Thus, in Figure 8(a), the measurements of the centre of gravity are shown for six different parts of the treatment, three on the left side (marked with L1, L2 and L3) and three on the right side (marked with R1, R2 and R3), which were performed alternatively (i.e. left side, right side, left side, etc.). In the graphs the x-y trajectories of the centre of gravity can be seen, and it can also be seen that there is a clear distinction between the treatment on the left side and the treatment on the right side.

The graphs in Figures 8(b) and 8(c) show similar results for two other users, although it can be seen that the second user (Figure 8(b)) leant further forward than the other users while performing the treatment (as shown by the position of the centre of gravity trajectories in the upper part of the graph). It can also be seen that the second user favoured the left leg with his or her weight, since the trajectories are towards the left side of the graph, and the third user (Figure 8(c)) favoured the right leg with his or her weight, since the trajectories are towards the right side of the graph.

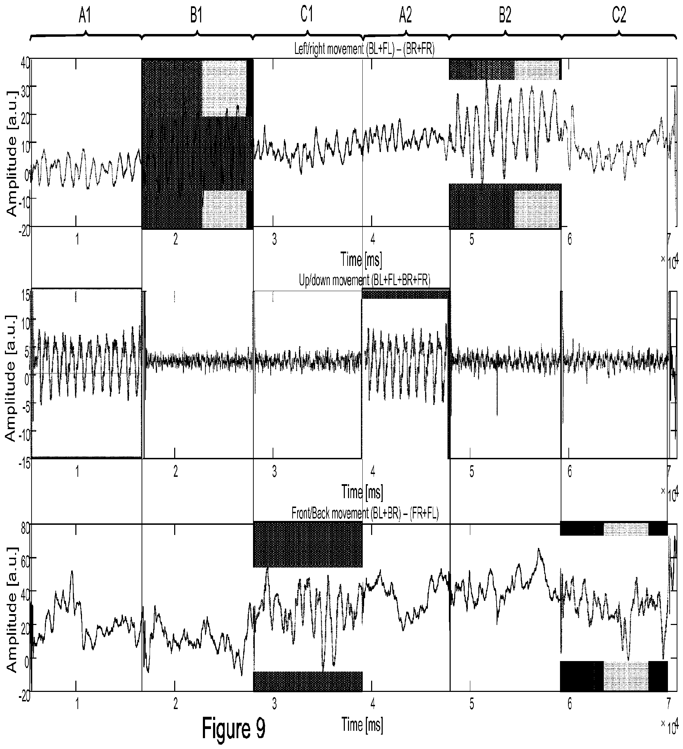

The graphs in Figure 9 illustrate how a direction of movement of a handheld personal care device could be determined from changes in the weight distribution in a predetermined area over time. That is, Figure 9 shows how left/right movements, up/down movements and front/back movements can be distinguished and identified from measurements of the weight distribution.

A weight distribution measurement apparatus 20 as shown in Figure 4 was used to collect measurements of the weight in the four quadrants 20 (front left, FL, front right, FR, back left, BL, back right, BR) as a user performed an up/down movement (time period A1), followed by a left/right movement (time period B1), followed by a front/back movement (time period C1), followed by another up/down movement (time period A2), followed by another left/right movement (time period B2), followed by another front/back movement (time period C2). The weight measurements have been combined in three different ways to determine if these movements can be identified.

Figure 9(a) shows the resulting amplitude from combining the weight measurements using the equation (BL + FL) - (BR + FR), and thus finds the difference between the weight measurements in the left half (BL + FL) and the right half (BR + FR). Figure 9(b) shows the resulting amplitude from combining the weight measurements using the equation BL + FL + BR + FR, and thus finds the changes in the total weight over time. Figure 9(c) shows the resulting amplitude from combining the weight measurements using the equation (BL + BR) - (FL + FR), and thus finds the difference between the weight measurements in the front half (FR + FL) and the back half (BR + BL).

The shaded blocks in each of Figures 9(a), 9(b) and (c) indicate the parts of the resulting amplitude that show a clear oscillation, and it can be seen that the equation used for Figure 9(a) is able to isolate/identify left/right movements in the time periods B1 and B2, the equation used for Figure 9(b) is able to isolate/identify up/down movements in the time periods A1 and A2, and equation used for Figure 9(c) is able to isolate/identify front/back movements in the time periods C1 and C2. It will be noted that the results obtained from Figure 9(c) is not as clear as the results from Figures 9(a) and 9(b), and this is due to the fact that the body is better at correcting or compensating for forward/backward movement than side to side or up and down movement. However, the use of additional signal filtering and/or frequency analysis can improve the classification of device movement.

It will be appreciated that the equations used to form the amplitudes in Figures 9(a)-(c) are merely exemplary and other equations could be used. It will also be appreciated that similar equations can be used for measurements of torque.

Based on the above described techniques to estimate the position of the handheld personal care device and movements of the device, the control unit 4 can provide guidance to the user 40 or operator of the device on the personal care activity. For example the control unit 4 could provide feedback or guidance on oral health care routines, cleansing routines and shaving habits based on the usage of the device on the right and left side of the face in combination with the periodic movement of the device.

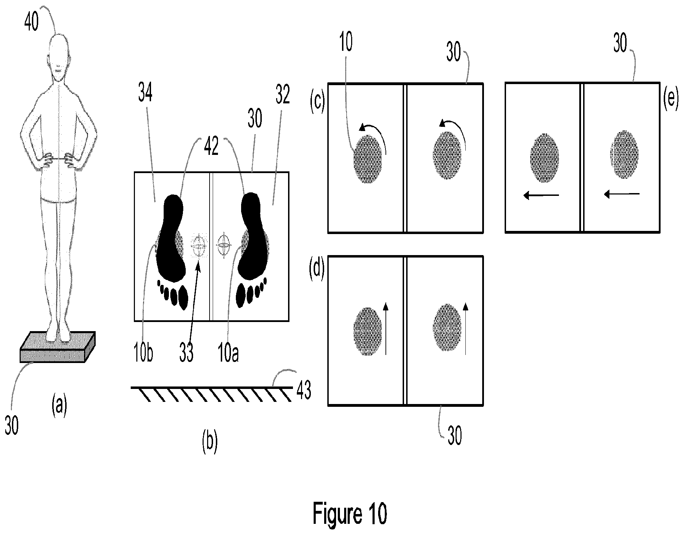

Figure 10 illustrates an exemplary mapping or predetermined relationship between the torque imparted and the position of a handheld personal care device relative to a user. In particular the torque imparted provides an indication of the orientation and/or rotation of the head and/or shoulders of the user 40.

Figure 10(a) shows a user 40 standing on a torque measurement apparatus 30 as described above with reference to Figure 5. Figure 10(b) shows a top view of the torque measurement apparatus 30 with the feet 42 of the user 40 shown positioned on the torque measurement apparatus 30. The user 40 is standing on the torque measurement apparatus 30 in front of a mirror 43. As in Figure 5, the torque measurement apparatus 30 comprises two halves 32, 34, each with a corresponding torque sensor 10a, 10b. In this embodiment, each torque sensor 10 measures torque around three orthogonal axes. The left foot 42 of the user 40 is on the left half 32 and the right foot is on the right half 34. It will be appreciated that halves 32, 34 have been labelled from the point of view of the user 40.

The scenario shown in Figure 10(c) corresponds to the user 40 having rotated the torso and head to the left side, which results in a torque in an anti-clockwise direction (when looking down onto the torque measurement apparatus 30). This rotation could occur when the user 40 is using the handheld personal care device on the right side of their face.

In Figure 10(d), the user 40 is using a handheld personal care device on the neck and has tilted the head back. This shifts some of the weight of the user 40 backwards (i.e. away from the mirror 43), and results in a torque in a direction that is downwards on the back side of the torque measurement apparatus 30 and upwards on the front side of the torque measurement apparatus 30 (i.e. the side nearest the mirror 43).

In Figure 10(e), the user 40 is using a handheld personal care device on the right side of the head and has turned the head/body to the right to better see that area in the mirror 43. This has shifted some of the weight of the user 40 to the right, and results in a torque in a direction that is downwards on the right side of the torque measurement apparatus 30 and upwards on the left side of the torque measurement apparatus 30.

Thus, in some embodiments, the predetermined relationship defines that the handheld personal care device is on the left side of the head of the user 40 if the measured torque imparted indicates that the user 40 is turned to the right, and that the handheld personal care device is on the right side of the head of the user 40 if the measured torque imparted indicates that the user 40 is turned to the left. In some embodiments, the predetermined relationship defines that the handheld personal care device is on the front side of the head of the user 40 if the measured torque imparted indicates that the user 40 is facing forwards.

As noted above, in some embodiments, measurements of weight and torque can be obtained and the measurements can be used together to estimate the position of the handheld personal care device relative to the user 40. In some embodiments, the measurements of the weight distribution can be assessed to estimate a position, and the measurements of the imparted torque can be assessed to estimate a position, and an overall estimate of the position can be determined from the two estimates. In other embodiments, the measurements of the weight distribution and the measurements of the torque imparted can be assessed together to estimate the position. In this case, the predetermined relationship can relate positions of the handheld personal care device to particular weight distributions and torque(s).

It will be appreciated that in some embodiments the control unit 4 can update the predetermined relationship over time based on usage of the handheld personal care device to improve the accuracy of the position estimation. For example the control unit 4 can make use of machine learning to improve the estimation of the position of the handheld personal care device and thus detection of the treatment area by analysing the previous usage of the device by the user 40 or the operator and updating a training set or data set used for position estimation.

In some embodiments, the control unit 4, or more generally the apparatus 2, can transmit information or data on the usage of the handheld personal care device, including information on the position of the handheld personal care device over time, to a computer (e.g. a remote server). The computer can assess the information or data for the handheld personal care device, or for multiple types of handheld personal care devices used by the user 40, or used by different users, to provide personalised treatment and/or personal care settings for the handheld personal care device. These settings (e.g. parameters that control the operation of a personal care component) can be provided to the apparatus 2 or the handheld personal care device to better personalise treatment and/or personal care activity.

There is therefore provided an improved technique for estimating the position of a handheld personal care device relative to a user. In particular, it will be appreciated that, although the improved technique may not necessarily provide the position with a high resolution (e.g. the technique provides a general region or area of the body, such as the neck or the left cheek, rather a specific point on the body), the technique provides that the position can be estimated without having to modify the handheld personal care device (so the technique can be used with conventional 'non-smart' handheld personal care devices) and without requiring the use of cameras or other complex processing technology.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method of estimating a position of a handheld personal care device relative to a user of the handheld personal care device, the method comprising:
obtaining one or both of (i) measurements of a weight distribution of the user in a predetermined area over time and (ii) measurements of a torque imparted to the predetermined area by one or both feet of the user over time; and
estimating the position of the handheld personal care device relative to the user using the obtained measurements and based on a predetermined relationship between the weight distribution and/or the torque imparted and the position of the handheld personal care device relative to the user.

2. A method as claimed in claim 1, wherein the predetermined relationship defines a respective characteristic of the weight distribution and/or the torque imparted for each of a plurality of positions of the handheld personal care device relative to the user.

3. A method as claimed in claim 1 or 2, wherein the predetermined relationship defines at least a first weight distribution and/or a first torque imparted for a first position of the handheld personal care device relative to the user and a second weight distribution and/or a second torque imparted for a second position of the handheld personal care device relative to the user.

4. A method as claimed in any one of the claims 1-3, wherein the position of the handheld personal care device relative to the user is a position of the handheld personal care device relative to the head of the user.

5. A method as claimed in claim 4, wherein the predetermined relationship defines that the handheld personal care device is on the left side of the head of the user if the measured weight distribution indicates that a centre of gravity of the weight distribution is in a right half of the predetermined area, and that the handheld personal care device is on the right side of the head of the user if the measured weight distribution indicates that the centre of gravity of the weight distribution is in a left half of the predetermined area.

6. A method as claimed in claim 4, wherein the predetermined relationship defines that the handheld personal care device is on the front side of the head of the user if the measured weight distribution indicates that a centre of gravity of the weight distribution is in or near a middle of the predetermined area.

7. A method as claimed in claim 4, wherein the predetermined relationship defines that the handheld personal care device is on or near the neck of the user if the measured weight distribution indicates that a centre of gravity of the weight distribution is in a rear half of the predetermined area.

8. A method as claimed in claim 4, wherein the predetermined relationship defines that the handheld personal care device is on the left side of the head of the user if the measured torque imparted indicates that the user is turned to the right, and that the handheld personal care device is on the right side of the head of the user if the measured torque imparted indicates that the user is turned to the left.

9. A method as claimed in claim 4, wherein the predetermined relationship defines that the handheld personal care device is on the front side of the head of the user if the measured torque imparted indicates that the user is facing forwards.

10. A method as claimed in any one of the claims 1-9, wherein the method further comprises the step of:
estimating a motion of the handheld personal care device using the obtained measurements.

11. A computer program product comprising a computer readable medium having computer readable code embodied therein, the computer readable code being configured such that, on execution by a suitable computer or processor, the computer or processor is caused to perform the method of any one of the claims 1-10.

12. An apparatus for estimating a position of a handheld personal care device relative to a user of the handheld personal care device, the apparatus comprising:
a control unit that is configured to:
obtain one or both of (i) measurements of a weight distribution of the user in a predetermined area over time and (ii) measurements of a torque imparted to the predetermined area by one or both feet of the user over time; and
estimate the position of the handheld personal care device relative to the user using the obtained measurements and based on a predetermined relationship between the weight distribution and/or the torque imparted and the position of the handheld personal care device relative to the user.

13. An apparatus as claimed in claim 12, wherein the predetermined relationship defines a respective characteristic of the weight distribution and/or the torque imparted for each of a plurality of positions of the handheld personal care device relative to the user.

14. An apparatus as claimed in claim 12 or 13, wherein the predetermined relationship defines at least a first weight distribution and/or a first torque imparted for a first position of the handheld personal care device relative to the user and a second weight distribution and/or a second torque imparted for a second position of the handheld personal care device relative to the user.

15. An apparatus as claimed in any one of the claims 12-14, further comprising one or both of:
a weight sensor for measuring the weight distribution of the user in the predetermined area; and
a torque sensor for measuring the torque imparted to the predetermined area by one or both feet of the user.
